(19) **Europäisches Patentamt**

**European Patent Office**

**Office européen des brevets**

(11) Veröffentlichungsnummer: **0 141 965**
A1

(12) **EUROPÄISCHE PATENTANMELDUNG**

(21) Anmeldenummer: **84110884.8**

(22) Anmeldetag: **12.09.84**

(51) Int. Cl.⁴: **G 01 F 1/704**, G 01 F 1/64,
A 61 M 5/14

(30) Priorität: **26.09.83 DE 3334805**

(43) Veröffentlichungstag der Anmeldung: **22.05.85**
**Patentblatt 85/21**

(84) Benannte Vertragsstaaten: **DE FR GB IT NL SE**

(71) Anmelder: **Siemens Aktiengesellschaft, Berlin und München Wittelsbacherplatz 2, D-8000 München 2 (DE)**

(72) Erfinder: **Prestele, Karl, Bismarckstrasse 21d, D-8520 Erlangen (DE)**

(54) **Verfahren und Vorrichtung zur Durchflussmessung kleiner Flüssigkeitsmengen.**

㊄ Die Erfindung bezieht sich auf ein Verfahren und eine Vorrichtung zur Durchflussmessung kleiner Flüssigkeitsmengen (7) durch ein dünnes rohrförmiges Element (5). Die Erfassung kleiner Flüssigkeitsströme (7) im Bereich von z.B. 1 - 500 µl/h ist besonders bei medizinischen Dosiergeräten von grosser Bedeutung. In das rohrförmige Element (5) werden gezielt Gasblasen (1) eingebracht, deren Durchmesser im wesentlichen gleich ist dem Innendurchmesser des rohrförmigen Elements (5). Der Flüssigkeitsstrom schiebt die Gasblase (1) vor sich her. An vorgegebenen Messpunkten (M1, M2), die in einem vorgegebenen Abstand voneinander entlang des rohrförmigen Elements (5) liegen, wird die Durchlaufzeit der Gasblasen (1) erfasst. Entsprechend der Durchlaufzeit kann ein Steuersignal (A) abgegeben werden, das zur Steuerung der Durchflussmenge verwendet wird. Das Verfahren und die Vorrichtung sind für elektrisch leitende Flüssigkeiten (7) geeignet, wobei die Änderung des elektrischen Widerstandes längs der Strömungsrichtung durch Blaseneintritt in die Messstrecke (M1,M2) erfasst wird.

Siemens Aktiengesellschaft    Unser Zeichen
Berlin und München           VPA 83 P 3261 E

**Verfahren und Vorrichtung zur Durchflußmessung kleiner Flüssigkeitsmengen**

Die Erfindung bezieht sich auf ein Verfahren zur Durchflußmessung kleiner Flüssigkeitsmengen, die durch ein rohrförmiges Element strömen, in welches eine Gasblase eingebracht wird, deren Durchlaufzeit zwischen zwei vorgegebenen, in Längsrichtung des rohrförmigen Elements beabstandeten Punkten gemessen wird, wobei der Durchmesser der Gasblase im wesentlichen gleich dem Innendurchmesser des rohrförmigen Elementes gewählt ist, und wobei aus der Durchlaufzeit die Durchflußmenge ermittelt wird. Die Erfindung bezieht sich auch auf eine Durchflußmeßvorrichtung zur Durchführung des Verfahrens.

Die Messung sehr kleiner Flüssigkeitsströme, vornehmlich zwischen 1 und 500 µl/h, stellt insbesondere bei Medikamentendosiergeräten ein schwer lösbares Problem dar. Deshalb ist es bisher nicht gelungen, eine wirklich befriedigende Funktionskontrolle für miniaturisierte Medikamentendosiergeräte zu realisieren. Eine solche Kontrolle ist jedoch dringend erforderlich, weil der Patient bei nicht erkannten Funktionsstörungen gefährdet sein kann. Funktionsstörungen sind am Medikamentendosiergerät selber möglich, z.B. durch Versagen von Bauteilen oder durch Batteriemängel, aber auch auf dem Dosierweg durch Verstopfung des engen Zuführungsschlauches oder -rohres mit Medikamentenflocken oder durch Zuwachsen der Austrittsöffnung für das Medikament. Die Forderung nach einer zuverlässigen Funktionskontrolle stellt sich bei implantierbaren Medikamentendosiergeräten noch verstärkt.

Wil 2 Rl / 28.02.1984

Ein Verfahren und eine Vorrichtung der eingangs genannten Art sind in der US-PS 3,739,636 beschrieben. Dort ist eine Durchfluß-Meßmethode angegeben, bei welcher zwei Detektoren in einem vorgegebenen Abstand zueinander angeordnet sind. Als Einrichtung zur Zuführung einer Gasblase ist dort entweder eine Nadel oder aber ein Dreiwegehahn eingesetzt, die jeweils mit einem Gasreservoir verbunden sind. Die Gasblase wird in das rohrförmige Element eingebracht und vom Strömungsfluß mitgeführt. Passiert die Gasblase den ersten Detektor, so wird eine Zeitmessung gestartet, die wieder unterbrochen wird, sobald die Gasblase den zweiten Detektor erreicht. Als Detektoren werden eine Lampe einerseits und eine Fotozelle andererseits verwendet. Diese Anordnung hat den Nachteil, daß dort, wo die Apperatur zur Durchflußmessung möglichst klein sein muß, wie z.B. auf dem medizinischen Anwendungsgebiet der Medikamentendosiergeräte, eine Lampe und eine Fotozelle zu sperrig und damit ungeeignet sind.

Ähnliche Verfahren und Vorrichtungen sind auch in der GB-Patentpublikation 2,083,612 und in der US-Patentschrift 3,621,715 beschrieben.

Ebenfalls mit der Messung kleiner Durchflußmengen befaßt sich die DE-PS 831 610. Dort wird zur Messung des Kraftstoffverbrauchs in einem Kraftfahrzeug eine Gasblase in ein Meßrohrstück eingebracht. Der Vorgang wiederholt sich zu fest vorgegebenen Zeiten. Zum Zwecke der Messung wird das Meßrohrstück mit zwei Absperrventilen vom Flüssigkeitsstrom abgetrennt. Mit Hilfe einer Skala wird festgestellt, wie weit die Gasblase in dem Röhrchen weitergewandert ist. Weiterhin wird dort angegeben, daß als Detektor für die Gasblase zwei sich im Röhrchen gegenüberliegende elektrische Kontakte geeignet sind. Ändert sich der Widerstand des Stromkreises, in welchem die Kontakte und eine Stromquelle liegen, so schließt ein Relais. Nachteilig bei dieser Anordnung ist, daß zur Auswertung der Durchflußmenge ein komplizierter handwerklicher Vorgang nötig ist, wie er z.B. in einem medizinischen Medikamentendosiergerät, welches implantiert ist, überhaupt nicht durchführbar wäre. Außerdem bedarf die Messung einer Anzeigeskala, die bei einem implantierbaren Dosiergerät nicht realisierbar ist.

Ebenfalls auf dem Sektor der Kraftstoffverbrauchsmessung ist die DE-OS 27 52 328 angesiedelt. Dort werden mit Hilfe einer ionisierenden ersten Elektrode Ionen erzeugt, die vom Strom des Kraftstoffes mitgerissen und an einer stromabwärts liegenden zweiten Elektrode wieder aufgefangen werden. Aus der Laufzeit der Ionen zwischen beiden Elektroden wird die Durchlaufzeit ermittelt. Diese Vorrichtung ist laut Angabe insbesondere für die Messung hoher Durchsätze geeignet. Das liegt daran, daß die Verschiebung der Ionen in Längsrichtung, d.h. in Strömungsrichtung, ausgenutzt wird. Bei niedrigen Durchflußraten oder kleinen Flüssigkeitsströmen würden die Ionen wieder rekombinieren, und es würde eine Verfälschung des Meßsignals eintreten.

In der DE-OS 22 18 459 wird ein Verfahren beschrieben, das die Durchflußmessung in einem hydraulischen System behandelt. Insbesondere geht es um die Messung des Massenflusses einer diskontinuierlichen Phase oder um die Messung des Durchflußvolumens einer Flüssigkeitsmischung, die eine leitende Flüssigkeit enthält. Mit Hilfe von zwei Elektroden, die mit dem Flüssigkeitsstrom in Berührung stehen, wird die elektrische Leitfähigkeit gemessen. Aus dieser Messung wird ein Signal gebildet, das eine Veränderung der Leitfähigkeit anzeigt.

0141965

- 4 -    VPA 83 P 3261 E

Diese Veränderung der Leitfähigkeit beruht auf einer Veränderung im Verhältnis der Bestandteile der Flüssigkeitsmischung. Aus den gemessenen Leitfähigkeitsänderungen wird über die Durchlaufzeit der Massenfluß ermittelt. Voraussetzung für die Messung ist, daß sich die Änderungen der Leitfähigkeit mit einer hohen Geschwindigkeit vollziehen. Sie müssen also auf Turbulenzen zurückzuführen sein. Auch bei diesem Meßverfahren werden also hohe Durchflußgeschwindigkeiten vorausgesetzt. Demgegenüber bezieht sich die vorliegende Erfindung auf die Messung und den Nachweis kleiner Flüssigkeitsmengen und geringer Durchflußgeschwindigkeiten in einem rohrförmigen Element kleinen Querschnitts.

Aufgabe vorliegender Erfindung ist es, ein Verfahren und eine Vorrichtung anzugeben, die speziell für den Nachweis sehr kleiner Durchflußmengen von Flüssigkeiten geeignet sind, und zwar insbesondere auf dem Gebiet medizinischer Dosiergeräte, bei welchem kleine und wenige Bauteile wünschenswert sind.

Diese Aufgabe wird bei dem Verfahren der eingangs genannten Art erfindungsgemäß dadurch gelöst, daß zur Durchflußmessung an einer elektrisch leitfähigen Flüssigkeit der elektrische Widerstand zwischen den beiden Punkten entlang der Längsrichtung des rohrförmigen Elements erfaßt wird, wobei sich zwischen den beiden Punkten ein relativ niedriger Widerstand ergibt, wenn sich keine Gasblase zwischen den beiden Punkten befindet, und wobei sich ein relativ hoher Widerstand ergibt, wenn sich eine Gasblase zwischen den beiden Punkten befindet.

Eine Vorrichtung zur Durchführung des Verfahrens geht aus von einer Durchflußmeßvorrichtung mit einem rohrförmigen Element, das von einer Flüssigkeit durchflossen ist, mit einer Einrichtung zur wiederholten Zuführung einer Gasblase in das rohrförmige Element und mit einer Einrichtung zur Erfassung des Durchlaufs der Gasblase zwischen zwei vorgegebenen Punkten des rohrförmigen Elements. Sie zeichnet sich erfindungsgemäß dadurch aus, daß an jedem der beiden Punkt entlang des rohrförmigen Elements eine Elektrode vorgesehen ist, und daß zwischen den beiden Elektroden eine Einrichtung zur Messung der Änderung des elektrischen Widerstands angeschlossen ist.

Vorteil des vorliegenden Verfahrens und der vorliegenden Vorrichtung ist es, daß diese speziell für den Anwendungsfall von kleinen Flüssigkeitsdurchsätzen durch enge rohrförmige Elemente wie Schläuche oder feste Röhren in einem medizinischen Dosiergerät geeignet sind. Durch das elektrische Meßverfahren werden große, sperrige Bauelemente, wie z.B. eine Lampe und eine Fotozelle vermieden. Gleichzeitig kommt man, aufgrund der elektrischen Leitfähigkeitsmessung entlang einer bestimmten Wegstrecke des Röhrchens, mit nur zwei Elektroden und deren Zuleitungen aus. Die strömende leitfähige Flüssigkeit kann dabei eine leichtflüssige oder eine zähflüssige Substanz sein.

Vom apparativen Aufbau her bringt eine weitere Ausgestaltung einen wesentlichen Vorteil mit sich. Bei leitenden Flüssigkeiten muß nicht über eine größere Bandbreite von dicht beieinander liegenden Widerstandswerten unterschieden werden, sondern nur zwischen den beiden Zuständen "leitend" und "nichtleitend". Eine zwischen den beiden Punkten in der Flüssigkeit sich befindliche Gasblase unterbricht nämlich die elektrische Stromleitung. Dies führt zu einer hohen Sicherheit des gewonnenen Ergebnisses. Fehlmessungen und Fehlinterpretationen der Messungen sind bei nur zwei möglichen Zuständen außerordentlich gering.

Ein weiterer wesentlicher Vorteil ergibt sich bei Einsatz der Vorrichtung in einem Medikamentendosiergerät, wenn ein Meßpunkt, d.h. eine Meßelektrode, das metallische Gehäuse dieses Dosiergerätes bildet. Dadurch wird der Meßpunkt elektrisch wirksam an die Katheterspitze eines an dem Dosiergerät befindlichen Katheters gelegt. Es kann überwacht werden, ob die Flüssigkeit auch tatsächlich am Katheterende austritt. Mit Hilfe der elektrischen Widerstandsmeßeinrichtung ist es dabei weiterhin möglich, eine Änderung des elektrischen Widerstandes an der Katheterspitze selbst zu erkennen. Solch eine Änderung kann beispielsweise durch Verstopfung der Katheterspitze oder durch Zuwachsen der Katheterspitze durch Körpergewebe verursacht werden. Störungen dieser Art können durch die Verlegung der Meßelektrode an das Gehäuse erkannt werden, und Gegenmaßnahmen, wie z.B. Austausch der Katheterspitze, können rechtzeitig eingeleitet werden.

In den beigefügten Figuren sind Ausführungsbeispiele der Erfindung dargestellt.

Es zeigen:

Fig. 1 eine Vorrichtung zur Messung kleiner Flüssigkeitsmengen,

Fig. 2 den typischen Ablauf eines Meßverfahrens, wie es Figur 1 zugrundeliegt, als Flußdiagramm für kontinuierlichen Durchlauf, und

Fig. 3 einen Längsschnitt durch ein rohrförmiges Element mit separater Elektrolysekammer zur Blasenerzeugung.

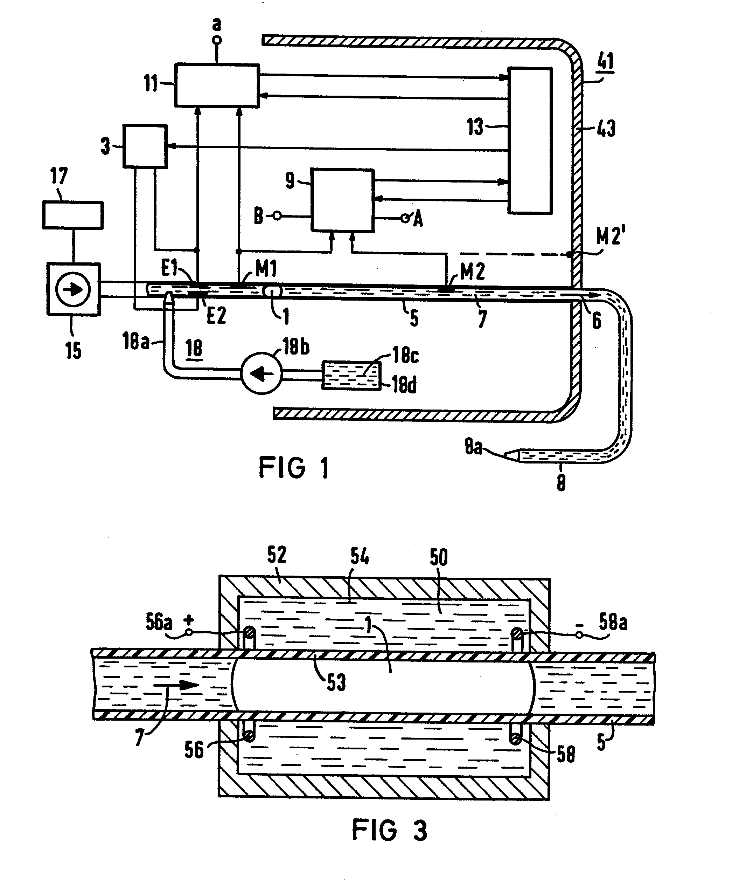

In Figur 1 sind mit E1 und E2 zwei Elektroden zur wiederholten Bildung einer einzelnen Gasblase 1 bezeichnet. Die
beiden Elektroden E1 und E2 sind elektrisch an einem
Gerät 3 zur Blasenerzeugung mittels Elektrolyse angeschlossen. Sie liegen im Verlauf eines rohrförmigen Elements oder Rohrstücks 5, z.B. eines Schlauches, durch
welches in Pfeilrichtung 6 eine Flüssigkeit 7 fließt. Die
Elektroden E1, E2 liegen sich im Rohrstück 5 gegenüber
und haben dabei Kontakt mit der Flüssigkeit 7. Die Elektroden E1, E2 können durch die Rohrwand geführte
metallische Stifte oder - wie gezeigt - an elektrische
Leitungen angeschlossene Metallplättchen sein. Sie werden
vom Gerät 3 nach Bedarf mit einer Gleichspannung beaufschlagt. Bei dem Rohrstück 5 kann es sich insbesondere um
einen Teil eines (nicht gezeigten) Katheters 8 mit Austrittsöffnung 8a eines implantierbaren Dosiergerätes
handeln, durch welchen ein Medikament in flüssiger Form
zu einer Applikationstelle in einem Patienten gepumpt
wird.

Stromabwärts von den Elektroden E1 und E2 liegt eine Meßstrecke, an deren Anfang sich eine erste Elektrode M1 und
an deren Ende sich eine zweite Elektrode M2 befindet.
Durch diese Meßelektroden M1, M2 sind zwei Punkte entlang
des Rohrstücks 5 vorgegeben. Auch hier sind die beiden
Elektroden M1, M2 so angebracht, daß sie in dauerndem
Kontakt zu der Flüssigkeit 7 stehen. Die Elektroden M1
und M2 sind über elektrische Zuleitungen an ein Gerät 9
zur Messung der elektrischen Leitfähigkeit angeschlossen.
Das Gerät 9 und die Elektroden M1, M2 bilden somit eine
elektrische Einrichtung zum Erfassen des Durchlaufs einer
Gasblase 1. An die Empfindlichkeit dieses Gerätes 9
brauchen keine hohen Anforderungen bezüglich der Meßgenauigkeit gestellt zu werden. Es reicht aus, wenn zwi-

schen dem Zustand "leitend" und dem Zustand "nichtleitend" unterschieden werden kann. Das Gerät 9 kann also eine nennenswerte Änderung des elektrischen Widerstands ohne weiteres nachweisen. Die Länge der Meßstrecke zwischen den beiden Elektroden M1, M2 richtet sich nach der zu erwartenden Strömungsgeschwindigkeit, der Art der Flüssigkeit 7 und dem Innendurchmesser des rohrförmigen Elements 5.

Die eine Elektrolyse-Elektrode E1 und die erste Meßelektrode M1 sind auch noch an ein Gerät 11 zur Störblasenerkennung angeschlossen. Das Gerät 11 zur Störblasenerkennung ermittelt ebenfalls über Widerstands- oder Leitfähigkeitsmessung, ob sich im Rohrstück 5 zwischen den beiden Elektroden E1 und M1 eine Gasblase befindet oder nicht. Eine solche Gasblase kann eine von den Elektrolyse-Elektroden E1, E2 erzeugte Gasblase 1 oder aber eine Störgasblase sein. Der Abstand der Elektroden E1, M1 kann kleiner gewählt sein als der Abstand der Meßelektroden M1, M2. Alternativ hierzu können die Elektroden E1 und M1 zu einer Elektrode zusammengefaßt sein. Dieser zusammengefaßten Elektrode gegenüber liegt dann E2.

Das Gerät 11 zur Erkennung störender Gasblasen kann somit ein Alarmsignal a abgeben. Dieses wird an ein (nicht gezeigtes) Alarmgerät weitergeleitet, welches bei Auftreten der störenden Gasblasen einen z.B. akustischen oder optischen Alarm gibt.

Eine Steuerungseinrichtung 13 übernimmt die Abfrage der Geräte 11 und 9, ob Leitfähigkeit zwischen den Elektroden E1 und M1 einerseits und M1 und M2 andererseits vorliegt oder nicht. Je nach Ergebnis dieser Abfrage leitet sie weitere Schritte ein, die später noch beschrieben werden.

- 9 -    VPA 83 P 3261 E

Das Rohrstück 5 ist, eventuell über einen nicht gezeigten Katheter, an eine Pumpe 15 angeschlossen. Die Pumpe 15 fördert die Flüssigkeit 7 aus einem Reservoir 17 heraus durch das Rohrstück 5. Am Ende des Rohrstücks 5 steht die Flüssigkeit 7 zur weiteren Verwendung bereit. Bei der Flüssigkeit 7 kann es sich z.B. um Insulinlösung handeln, und bei der weiteren Verwendung um die dosierte Zugabe in den Körper eines Patienten.

Wichtig bei der Vorrichtung nach Figur 1 ist, daß der Durchmesser der erzeugten Gasblase 1 nicht wesentlich kleiner, also im wesentlichen immer gleich dem Innendurchmesser des Rohrstücks 5 ist. Das heißt, daß die Flüssigkeit 7 nicht an der Gasblase 1 vorbeiströmen kann, sondern diese vor sich herschiebt. Bei den hier betrachteten Volumenströmen von ca. 1 bis 500 µl/h bei einem Rohrinnendurchmesser von 0,1 bis 2,0 Millimetern kann davon ausgegangen werden, da es nicht zu Turbulenzen oder anderen Strömungsunregelmäßigkeiten kommt. Es kann somit von einem gleichmäßigen (laminaren) Strömungsvorgang gesprochen werden.

Aufgrund der genannten niedrigen Strömungsgeschwindigkeiten ist auch das Einbringen oder Einimpfen der Gasblase 1 nicht problematisch. Es kommt zu keinem Wegspülen vor dem Erreichen des gewünschten Durchmessers der Gasblase 1. Zum Erzielen einer ausreichend großen Gasblase 1 ist es angebracht, der Elektrode E1, an der sich die Gasblase 1 bildet, eine spezielle Form zu geben. So stellt z.B. eine langgestreckte Elektrode genügend Fläche zur Verfügung, um die Strecke zwischen den Elektroden E1, E2 nicht vor Erreichen der nötigen Blasenstärke zu isolieren. Es sind auch speziell gekrümmte Elektrodenformen denkbar, die die Form der Gasblase 1 mitgestalten.

Wie bereits erwähnt, kann die Gasblase 1 durch Elektrolyse erzeugt werden. Enthält die Flüssigkeit 7 z.B. Wasser, so wird dieses bei ausreichend hoher Spannung an den Elektroden El und E2 in Wasserstoff ($H_2$) und Sauerstoff ($O_2$) zerlegt. Bei nicht elektrolysefähiger Flüssigkeit 7 kann die Gasblase 1 stattdessen von einer Einrichtung 18 aus einem in das rohrförmige Element 5 einmündenden Schlauch- oder Rohrstück 18a gesteuert zugesetzt werden. Das Schlauch- oder Rohrstück 18a wird von einer Pumpe 18b mit Gas 18c versorgt, welches in einem Reservoir 18d bevorratet ist.

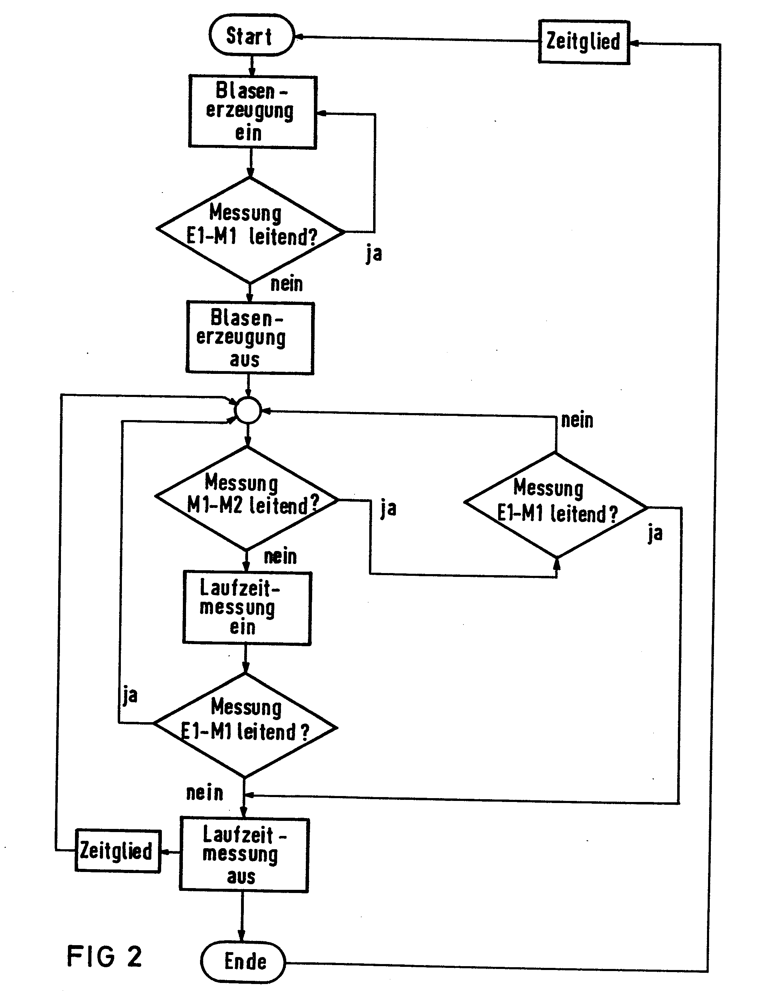

Ein typischer Ablauf eines Meßverfahrens mit der in Figur 1 gezeigten Vorrichtung sieht nach Figur 2 folgendermaßen aus: Die Steuereinrichtung 13 gibt ein Startsignal an das Gerät 3 zur Blasenerzeugung. Im gezeigten Ausführungsbeispiel ist dieses ein Elektrolysegerät, welches an die Elektroden El und E2 eine Gleichspannung legt. Daraufhin bilden sich an mindestens einer der Elektroden, z.B. an der Elektrode El, kleine Gasbläschen. Im zeitlichen Verlaufe vereinigen sich die kleinen Bläschen zu einer größeren Gasblase 1, die nach einer gewissen Zeit den Durchmesser des Rohrstücks 5 ausfüllt und in Pfeilrichtung 6 wandert oder bei Flüssigkeitsstillstand im Bereich der Elektroden El, E2 verharrt. Als Folge davon ist die Elektrode El von der Elektrode Ml elektrisch isoliert. Die Steuerungseinrichtung 13 fragt nun in periodischen vorgegebenen Zeitabständen ab, ob die Strecke zwischen den Elektroden El und Ml "leitend" oder "nichtleitend" ist. Ist sie "leitend", ist die Gasblase 1 noch nicht ausreichend gewachsen, um den Querschnitt des Rohrstücks 5 auszufüllen, und das Gerät 3 zur Gasblasenerzeugung bleibt eingeschaltet. Ist die Strecke "nichtleitend", gibt die Steuerungseinrichtung 13 ein Stopsignal an das Gerät 3 zur Blasenbildung, welches abschaltet.

- 11 -    VPA 83 P 3261 E

Nach diesem Vorgang fragt die Steuerungseinrichtung 13 in vorgegebenen, periodischen Zeitabständen die Meßstrecke zwischen den Elektroden M1 und M2 darauf ab, ob sie "leitend" oder "nichtleitend" ist. Ist die Meßstrecke M1, M2 "leitend" (relativ niedriger Widerstand), wird durch Abfrage der Strecke E1 - M1 auf Leitfähigkeit geprüft, ob sich die Gasblase 1 noch vor der Meßstrecke M1 - M2 befindet. Ist dies der Fall, wird die Steuerungseinrichtung 13 erneut die Meßstrecke M1 - M2 auf "nichtleitend" abfragen. Diese Abfrageschleife wird solange wiederholt, bis die erzeugte Gasblase 1 in die Meßstrecke M1 - M2 eingetreten ist. Sobald die entsprechende Abfrage positiv ist, sobald also die Meßstrecke M1 - M2 "nichtleitend" ist (relativ hoher Widerstand), wird die Steuerungseinrichtung 13 eine Laufzeitmessung starten. Eine Einrichtung zur Laufzeitmessung ist im Gerät 9 in Figur 1 mit enthalten. Das Gerät 9 gibt ein Ausgangssignal A ab, das ein Maß für die Laufzeit der Gasblase 1 auf der Meßstrecke M1 - M2 und damit für die Durchflußmenge ist.

Von der Pumpe 15 her können u. U. Störblasen gefördert werden, z.B. aufgrund einer Verstopfung im Ausgangskanal oder weil das Reservoir 17 bereits geleert ist. Um zu kontrollieren, ob solche Störblasen in die Meßstrecke M1 - M2 eintreten, fragt die Steuerungseinrichtung 13 weiterhin periodisch, d.h. in vorgegebenen Zeitabständen, die Strecke E1 - M1 auf Leitfähigkeit ab.

a) Ist die Strecke E1 - M1 nichtleitend, muß eine Störblase in diese Strecke E1 - M1 eingetreten sein. In diesem Fall wird die laufende Messung von der Steuerungseinrichtung 13 unterbrochen und auf Null zurückgesetzt. Gleichzeitig wird die Störblase so behandelt, als ob sie eine erzeugte Gasblase 1 ist. Die Steuerungseinrichtung 13 schaltet also bis zum Signal

- 12 -    VPA 83 P 3261 E

"Blasenerzeugung Stop" zurück. Diese Rückschaltung erfolgt direkt nach der Unterbrechung und Nullstellung der Laufzeit.

b) Bleibt die Strecke E1 - M1 jedoch leitend, d.h. ist keine Störblase in die Strecke E1 - M1 eingetreten, so wird geprüft, ob sich die Gasblase 1 noch auf der Meßstrecke M1 - M2 befindet. Die Steuerungseinrichtung 13 schaltet also wieder zurück auf den Zustand nach "Blasenbildung Stop", im Gegensatz zum Fall a) allerdings, ohne vorher die Messung zu unterbrechen.

Diese Schleife wird solange durchlaufen, bis sowohl die Meßstrecke M1 - M2 als auch die Strecke E1 - M1 "leitend" ist. Dann wird von der Steuerungseinrichtung 13 die Laufzeitmessung gestoppt, und das Ergebnis wird als Ausgangssignal A zur weiteren Verwendung weitergegeben. Die weitere Verwendung kann z.B. zu einer Veränderung der Pumpgeschwindigkeit der Pumpe 15 führen. Das Ausgangssignal A kann aber auch einen akustischen und/oder optischen Alarm auslösen. Die weitere Verwendung kann auch nur aus einer Umrechnung auf die Durchflußmenge und deren Anzeige bestehen, was z.B. über induktive Signalkupplung oder Funk bei einem implantierten Gerät vonstatten gehen kann.

In einer weiteren Ausgestaltung des Verfahrens können die Störblasen bei ihrer Erfassung registriert werden, und ab einer gewissen Häufigkeit oder ab einem Grenzwert der Volumenprozente von Gasblasen 1 in der Flüssigkeit 7 kann durch ein Signal B optischer und/oder akustischer Alarm ausgelöst werden.

Nach dem Stoppen der Laufzeit ist der Meßvorgang abgeschlossen, und die Steuerungseinrichtung 13 kann über ein einstellbares Zeitglied 21 wieder an den Anfangspunkt zurückgesetzt werden. Dann gibt sie wieder ein Startsignal an das Gerät 3 zur Blasenbildung ab.

Es ist vorteilhaft, das rohrförmige Element 5, welches die Meßstrecken M1 - M2, E1 - M1 enthält, aus einem formbeständigen, nicht biegsamen Material zu fertigen. Dies gewährt konstante Volumenverhältnisse innerhalb der Meßstrecken M1 - M2, E1 - M1.

In einer weiteren Ausgestaltung der Vorrichtung sind ihre sämtlichen Komponenten in einem Gehäuse 41 untergebracht. Dies trifft vor allem bei implantierbaren Geräten zu. Es kann dann zweckmäßig sein, einen größeren Teil des Dosierweges zu überwachen. Speziell eine Kontrolle, ob an der Austrittsöffnung für die Flüssigkeit 7 noch ausreichender Durchlauf herrscht, ist angebracht.

Dieses kann gemessen werden, wenn die Meßelektrode M2 an eine Gehäusewand 43 gelegt wird, wobei die Gehäusewand elektrisch leitend ist. Die ist gestrichelt und als Meßelektrode M2' gezeigt. Die Leitfähigkeitsmessung überprüft dann den gesamten Weg von der Austrittsöffnung bis zur Meßelektrode M1 auf Vorhandensein der Gasblase 1. Der elektrische Widerstand des Gewebes zwischen Gehäusewand 43 und der Austrittsöffnung 8a für die Flüssigkeit 7 kann dabei gegenüber dem elektrischen Widerstand der Gasblase 1 im Rohrstück 5 vernachlässigt werden.

Die Figur 3 zeigt einen Längsschnitt durch das rohrförmige Element 5, dem hier eine separate Elektrolysekammer 50 zur Erzeugung der Gasblase 1 zugeordnet ist. Die Elektrolysekammer 50 ist zylindrisch ausgebildet und zentral um das rohrförmige Element 5 herum angeordnet. Die Elektrolysekammer 50 wird gebildet durch eine zylindrische Umhüllung 52, die diese vom Außenraum abgrenzt. Nach innen wird die Elektrolysekammer 50 abgegrenzt durch einen Abschnitt 53 des rohrförmigen Elementes 5. Die Elektrolysekammer 50 ist mit einem Elektrolyten 54, z.B. mit NaOH, gefüllt. Der in der Elektrolysekammer 50 untergebrachte Elektrolyt 54 kann hierbei als Gasreservoir angesehen werden. Zwei ringförmige Elektroden 56, 58 verlaufen innerhalb der Elektrolysekammer 50 konzentrisch um das rohrförmige Element 5 herum. Die Elektroden 56, 58 sind mit elektrischen Anschlüssen 56a bzw. 58a für eine Gleichspannung verbunden, wobei diese Anschlüsse 56a, 58a aus der Elektrolysekammer 50 herausgeführt sind.

Der Rohrabschnitt 53 ist als eine gaspermeable Wand ausgeführt. Sie kann z.B. aus einem Stück Silikonkautschuk bestehen. Sie trennt das Innere des rohrförmigen Elementes 5 von der Elektrolysekammer 50. Im gezeigten Beispiel besteht das gesamte rohrförmige Element 5 aus einem Silikonkautschuk-Schlauch. Stattdessen könnte auch eigens im Bereich der Elektrolysekammer 50 ein Zwischenstück als die gasdurchlässige Wand eingearbeitet sein.

An die Elektroden 56, 58 wird eine elektrische Gleichspannung angelegt, woraufhin in der Elektrolysekammer 50 eine Gasbildung einsetzt. Durch Diffusion durch den gaspermeablen Abschnitt 53 hindurch gelangt das Gas in das Innere des rohrförmigen Elementes 5. Es bildet sich dort zur Gasblase 1 aus.

Bei dieser Ausgestaltung der Gasblasenbildung durch Elektrolyse und Diffusion ist von Vorteil, daß die strömende Flüssigkeit 7 nicht elektrolysefähig zu sein braucht, und

- 15 -     VPA 83 P 3261 E

daß sie durch den elektrolytischen Prozeß nicht beeinträchtigt wird. Der Elektrolyt 54 und das Material der Elektrode 56, 58 sollten in vorteilhafter Weise für eine möglichst ergiebige Gasblasenbildung aufeinander abgestimmt sein.

14 Patentansprüche
3 Figuren

Patentansprüche

1. Verfahren zur Durchflußmessung kleiner Flüssigkeitsmengen, die durch ein rohrförmiges Element (5) strömen,
in welches eine Gasblase (1) eingebracht wird, deren
Durchlaufzeit zwischen zwei vorgegebenen, in Längsrichtung des rohrförmigen Elements (5) beabstandeten Punkten
(M1, M2) gemessen wird, wobei der Durchmesser der Gasblase (1) im wesentlichen gleich dem Innendurchmesser des
rohrförmigen Elementes (5) gewählt ist, und wobei aus der
Durchlaufzeit die Durchflußmenge ermittelt wird,   d a -
d u r c h g e k e n n z e i c h n e t ,   daß zur Durchflußmessung an einer elektrisch leitfähigen Flüssigkeit
der elektrische Widerstand zwischen den beiden Punkten
(M1, M2) entlang der Längsrichtung des rohrförmigen Elements (5) erfaßt wird, wobei sich zwischen den beiden
Punkten (M1, M2) ein relativ niedriger Widerstand ergibt,
wenn sich keine Gasblase (1) zwischen den beiden Punkten
(M1, M2) befindet, und wobei sich ein relativ hoher Widerstand ergibt, wenn sich eine Gasblase (1) zwischen den
beiden Punkten (M1, M2) befindet.

2. Verfahren nach Anspruch 1,   d a d u r c h   g e -
k e n n z e i c h n e t ,   daß das rohrförmige Element
(5) auf den Durchfluß störender Gasblasen (1) überwacht
wird, und daß bei Erkennung störender Gasblasen (1) die
Messung der Durchlaufzeit wiederholt wird.

3. Verfahren nach Anspruch 2,   d a d u r c h   g e -
k e n n z e i c h n e t ,   daß bei der Erkennung einer
störenden Gasblase (1) die laufende Messung der Durchlaufzeit unterbrochen wird, und daß der Erkennungszeitpunkt dieser störenden Gasblase (1) als neuer Startzeitpunkt für die neue Messung der Durchlaufzeit herangezogen
wird.

- 17 -    VPA 83 P 3261 E

4. Verfahren nach einem der Ansprüche 1 bis 3,  d a -
d u r c h   g e k e n n z e i c h n e t ,  daß aus der
Durchlaufzeit ein Steuersignal (s) abgeleitet wird, und
daß das Steuersignal (s) zum Steuern einer Fördereinrichtung (15) für die Flüssigkeit (7) vorgesehen ist.

5. Durchflußmeßvorrichtung zur Durchführung des Verfahrens nach Anspruch 1 mit einem rohrförmigen Element
(5), das von einer Flüssigkeit (7) durchflossen ist,
mit einer Einrichtung (3, El, E2) zur wiederholten Zuführung einer Gasblase (1) in das rohrförmige Element
(5), und mit einer Einrichtung (9, Ml, M2) zur Erfassung
des Durchlaufs der Gasblase (1) zwischen zwei vorgegebenen Punkten (Ml, M2) des rohrförmigen Elements (5),
d a d u r c h   g e k e n n z e i c h n e t , daß an
jedem der beiden Punkte (Ml, M2) entlang des rohrförmigen
Elements (5) eine Elektrode vorgesehen ist, und daß zwischen den beiden Elektroden eine Einrichtung (9) zur
Messung der Änderung des elektrischen Widerstands angeschlossen ist.

6. Durchflußmeßvorrichtung nach Anspruch 5,  d a -
d u r c h   g e k e n n z e i c h n e t ,  daß die
Einrichtung (9) zur Messung der Widerstandsänderung so
aufgebaut ist, daß sie lediglich zwischen einem Zustand
"leitend" und einem Zustand "nichtleitend" diskriminiert.

7. Durchflußmeßvorrichtung nach einem der Ansprüche
5 oder 6,  d a d u r c h   g e k e n n z e i c h -
n e t ,  daß die Einrichtung zur Zuführung der Gasblase (1) ein Elektrolysegerät (3) umfaßt, das die
Gasblase (1) durch Elektrolyse der zu messenden Flüssigkeit (7) erzeugt.

0141965

- 18 -    VPA 83 P 3261 E

8. Durchflußmeßvorrichtung nach einem der Ansprüche
5 bis 7, d a d u r c h  g e k e n n z e i c h n e t ,
daß das rohrförmige Element (5) in einem elektrisch
leitfähigen Gehäuse (41) angeordnet ist, wobei die Austrittsöffnung des rohrförmigen Elements (5) außerhalb
des Gehäuses (41) in einer elektrisch leitenden Umgebung
liegt, und daß das Gehäuse (41) gleichzeitig als eine
der beiden Meßelektroden (M1, M2) für die Einrichtung (9)
zur Messung der Änderung des elektrischen Widerstands
vorgesehen ist.

9. Durchflußmeßvorrichtung nach einem der Ansprüche
5 bis 8, d a d u r c h  g e k e n n z e i c h n e t ,
daß ein Gerät (11) zur Erkennung in dem rohrförmigen
Element (5) befindlicher störender Gasblasen (1) vorgesehen ist.

10. Durchflußmeßvorrichtung nach Anspruch 9,  d a -
d u r c h  g e k e n n z e i c h n e t ,  daß das
Gerät (11) zur Erkennung störender Gasblasen (1) aus
einem Gerät zur Widerstandsmessung und aus zwei Elektroden (E1, M1) besteht, die in kurzem Abstand zueinander
am rohrförmigen Element (5) angeordnet und die an das
Gerät zur Widerstandsmessung (9) angeschlossen sind.

11. Durchflußmeßvorrichtung nach einem der Ansprüche
9 oder 10, d a d u r c h  g e k e n n z e i c h -
n e t ,  daß das Gerät (11) zur Erkennung störender
Gasblasen (1) und zur Abgabe eines Alarmsignals (a)
vorgesehen ist.

12. Durchflußmeßvorrichtung nach einem der Ansprüche
5 bis 11, d a d u r c h  g e k e n n z e i c h n e t ,
daß die Einrichtung (9) zur Erfassung der Durchlaufszeit mit einem Gerät (13) verbunden ist, welches zum Steuern des Durchflusses der Flüssigkeit (7) dient.

13. Durchflußmeßvorrichtung nach einem der Ansprüche 5 bis 12, ausgenommen Anspruch 7, d a d u r c h   g e - k e n n z e i c h n e t ,   daß die Einrichtung zur Zuführung der Gasblase (1) eine elektrolytische Zersetzungsvorrichtung (50) ist, die einen Elektrolyten (54) enthält, der außerhalb des rohrförmigen Elementes (5) bevorratet ist.

14. Durchflußmeßvorrichtung nach Anspruch 13, d a - d u r c h   g e k e n n z e i c h n e t ,   daß der Elektrolyt (54) um das rohrförmige Element (5) herum angeordnet ist, wobei dieses rohrförmige Element (5) zumindest im Bereich des Elektrolyten (54) für das bei der Elektrolyse entstehende Zersetzungsgas durchlässig ist (Figur 3).

FIG 1

FIG 3

0141965

```
  ┌─────────┐                    ┌──────────┐
  │  Start  │◄──────────────────│ Zeitglied │◄────────────┐
  └─────────┘                    └──────────┘              │
       │                                                    │
       ▼                                                    │
  ┌─────────┐                                               │
  │ Blasen- │                                               │
  │erzeugung│◄────────┐                                     │
  │   ein   │         │                                     │
  └─────────┘         │                                     │
       │              │                                     │
       ▼              │ ja                                  │
   ╱ Messung ╲────────┘                                     │
  ◄ E1-M1 leitend? ►                                        │
   ╲         ╱                                              │
       │ nein                                               │
       ▼                                                    │
  ┌─────────┐                                               │
  │ Blasen- │                                               │
  │erzeugung│                                               │
  │   aus   │                                               │
  └─────────┘                                               │
       │         ┌────────────────────────── nein          │
       ▼         ▼                            │             │
      (○)◄───────────────────────┐           │             │
       │                         │           │             │
       ▼                         │           │             │
   ╱ Messung ╲ ja                │     ╱ Messung ╲         │
  ◄ M1-M2 leitend? ►─────────────│────► E1-M1 leitend? ►ja─┘
   ╲         ╱                    │     ╲         ╱
       │ nein                     │
       ▼                          │
  ┌─────────┐                     │
  │Laufzeit-│                     │
  │ messung │                     │
  │   ein   │                     │
  └─────────┘                     │
       │                          │
       ▼                          │
   ╱ Messung ╲ ja                 │
  ◄ E1-M1 leitend? ►──────────────┘
   ╲         ╱
       │ nein ◄──────────────────────────────────┐
       ▼                                          │
  ┌─────────┐      ┌──────────┐                  │
  │Laufzeit-│◄─────│ Zeitglied │                 │
  │ messung │      └──────────┘                  │
  │   aus   │                                     │
  └─────────┘                                     │
       │                                          │
       ▼                                          │
  ┌─────────┐                                     │
  │   Ende  │─────────────────────────────────────┘
  └─────────┘
```

FIG 2

Europäisches
Patentamt

**EUROPÄISCHER RECHERCHENBERICHT**

Nummer der Anmeldung

EP 84 11 0884

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-3 621 715 (SÖDERKVIST u.a.) <br> * Spalte 3, Zeilen 9-38; Spalte 3, Zeilen 3-67; Spalte 4, Zeile 48 - Spalte 5, Zeile 14; Spalte 5, Zeilen 50-53; Spalte 9, Zeile 19 - Spalte 10, Zeile 2; Spalte 10, Zeilen 22-27; Figuren * <br> --- | 1-3,9, 11,12 | G 01 F 1/70 <br> G 01 F 1/64 <br> A 61 M 5/14 |
| A | US-A-3 739 636 (A.A. VERSACI u.a.) <br> * Zusammenfassung; Figuren; Spalte 1, Zeilen 21-33; Spalte 2, Zeile 49 - Spalte 4, Zeile 68 * <br> --- | 1 | |
| A | GB-A-2 083 612 (CASSWELL) <br> * Zusammenfassung; Figuren; Seite 1, Zeilen 32-65; Seite 1, Zeilen 99-123; Seite 2, Zeilen 52-84 * <br> --- | 1 | RECHERCHIERTE SACHGEBIETE (Int. Cl.4) |
| A | DE-C- 831 610 (SCHERENBERG) <br> ----- | 1 | G 01 F <br> A 61 M |

Der vorliegende Recherchenbericht wurde für alle Patentansprüche erstellt.

| Recherchenort <br> DEN HAAG | Abschlußdatum der Recherche <br> 28-12-1984 | Prüfer <br> NUIJTEN E.M. |
|---|---|---|